# EUROPÄISCHE PATENTSCHRIFT

(11) **EP 3 120 672 B1**
(45) Veröffentlichungstag und Bekanntmachung des Hinweises auf die Patenterteilung: **25.12.2019**
(21) Anmeldenummer: 15711447.1
(22) Anmeldetag: 11.03.2015
(51) Int. Cl.: H05B 37/02, G06F 3/01

(54) **SYSTEM ZUM ANSTEUERN VON VERBRAUCHERN EINER HAUSHALTSLEITTECHNIK MITTELS MUSKELIMPULSEN WENIGSTENS EINES BENUTZERS UND ENTSPRECHENDES VERFAHREN**
SYSTEM FOR ACTUATING LOADS OF A DOMESTIC CONTROL APPARATUS BY MEANS OF MUSCLE IMPULSES OF AT LEAST ONE USER AND CORRESPONDING METHOD
SYSTÈME POUR ACTIVER DES CONSOMMATEURS D'UNE INSTALLATION DOMOTIQUE AU MOYEN D'IMPULSIONS MUSCULAIRES D'AU MOINS UN UTILISATEUR ET PROCÉDÉ CORRESPONDANT

(30) Priorität: 17.03.2014 DE 102014204889
(43) Veröffentlichungstag der Anmeldung: 25.01.2017
(73) Patentinhaber: Zumtobel Lighting GmbH, 6850 Dornbirn (AT)
(72) Erfinder: JOPPI, Rene, 6850 Dornbirn (AT); MAYR, Gregor, 6974 Gaißau (AT)
(74) Vertreter: Kiwit, Benedikt
(86) Internationale Anmeldenummer: PCT/EP2015/055034
(87) Internationale Veröffentlichungsnummer: WO 2015/140020

(56) Entgegenhaltungen:
- EP-A1- 1 408 443
- WO-A1-2010/079388
- US-A1- 2008 265 799
- US-A1- 2014 049 417

## Beschreibung

Die Erfindung betrifft ein System zum Ansteuern von Verbrauchern einer Haushaltsleittechnik, insbesondere von Leuchten, sowie ein Verfahren zum Ansteuern von Verbrauchern einer Hausleittechnik, insbesondere von Leuchten.

Aus dem Stand der Technik ist es bekannt, Leuchten vor allem über Taster und Schalter anzusteuern, insbesondere Licht über Taster und Schalter ein- und auszuschalten. Ferner sind aus dem Stand der Technik Lichtansteuerungen bekannt, die beispielsweise ein Hoch- und Runterdimmen von Licht, ein Konfigurieren und ein Auswählen verschiedener Lichtszenen sowie das Einstellen der Farbtemperatur von Licht ermöglichen. Solche Lichtansteuerungen können als Bediengeräte mit mehreren Tastern, wie beispielsweise das unter dem Namen "Circle Kit" bekannte Bediengerät der Anmelderin, oder als Bediengerät mit einem Touchdisplay, wie beispielsweise das unter dem Namen "Emotion Touch" bekannte Bediengerät der Anmelderin, ausgebildet sein.

Obwohl die Benutzerschnittstellen (user interfaces) solcher Bediengeräte eine große Weiterentwicklung zu den herkömmlichen Tastern und Schaltern darstellen, können sie mehr und mehr die modernen Anforderungen an eine intuitive Lichtansteuerung beziehungsweise an eine intuitive Ansteuerung der Leuchten eines Beleuchtungssystems nicht erfüllen. So werden Beleuchtungssysteme mit stetig steigenden Einstellungsmöglichkeiten entwickelt, wie beispielsweise Beleuchtungssysteme, bei denen die Helligkeit und die Farbtemperatur eingestellt, einzelne Leuchten oder einzelne Gruppen von Leuchten ausgewählt und bei denen verschiedene Lichtszenen konfiguriert und ausgewählt werden können.

Bei der Nutzung von herkömmlichen Bediengeräten für die Ansteuerung der Leuchten der aus dem Stand der Technik bekannten Beleuchtungssysteme kann der stetig wachsende Bedarf an die Einstellungsmöglichkeiten nur durch zwei Wege gelöst werden. Ein erster Weg wäre, Bediengeräte zu verwenden, die mehr Tasten und/oder mehr Eingabefelder aufweisen. Dadurch wird ein solches Bediengerät allerdings unübersichtlich. Ferner leidet dann die Intuitivität bei der Bedienung eines solchen Bediengerätes, sodass es für neue Anwender schwer bedienbar ist. Ein zweiter Weg wäre, Bediengeräte zu verwenden, die zusätzliche Untermenüs in der Menüführung aufweisen. Auch durch die Verwendung solcher Untermenüs wird die Bedienung unübersichtlich und die entsprechenden durch die Untermenüs eingefügten Funktionen müssen umständlich gesucht werden.

Nachteilig bei der Nutzung von aus dem Stand der Technik bekannten Bediengeräten mit klassischen Bedienelementen für die Ansteuerung der Leuchten eines Beleuchtungssystems ist daher, dass die Einstellungsmöglichkeiten der Leuchten beschränkt sind, oder, dass einzelne Einstellungen der Leuchten nur auf kompliziertem Wege vorgenommen werden können.

Bei der Ansteuerung der Leuchten von bekannten Beleuchtungssystemen, die für die Beleuchtung eines vorzugsweise größeren Raumes verwendet werden, werden insbesondere komplexere Bediengeräte verwendet, die aber in dem Raum oder in einem Raumsegment nur einmal oder maximal zweimal vorhanden sind. Nachteilig dabei ist, dass eine Ansteuerung der Leuchten von bekannten Beleuchtungssystemen durch mehrere Benutzer gleichzeitig nur schwer realisierbar ist. Dabei befinden sich die Bediengeräte in räumlicher Distanz zu den Benutzern, das heißt, dass sich die Benutzer jedes Mal zum Bediengerät bewegen müssen. Beispielsweise ist es bei der Verwendung von solchen in geringer Anzahl bereitgestellten Bediengeräten auch nicht möglich, dass mehrere Benutzer gleichzeitig jeweils die Leuchte über ihrem Arbeitsplatz ansteuern. Dadurch, dass ein einzelnes Bediengerät für die Ansteuerung einer großen Anzahl von Leuchten verwendet wird, ist es auch sehr umständlich die richtige Leuchte am Bediengerät auszuwählen. Das heißt, dass mit heutigen Bedienkonzepten eine intuitive und vor allem individuelle Ansteuerung der Leuchten eines herkömmlichen Beleuchtungssystems nur schwer realisierbar und in den meisten Situationen gar nicht möglich ist.

Aus dem Stand der Technik ist ferner bekannt, die Verbraucher eines Verbrauchersystems mit Hilfe von optisch erfassten Gesten anzusteuern. Bei dieser bekannten Gestenansteuerung werden die von einem Benutzer durchgeführten Gesten mit Hilfe einer Kamera erfasst und entsprechend ausgewertet und dann erkannt.

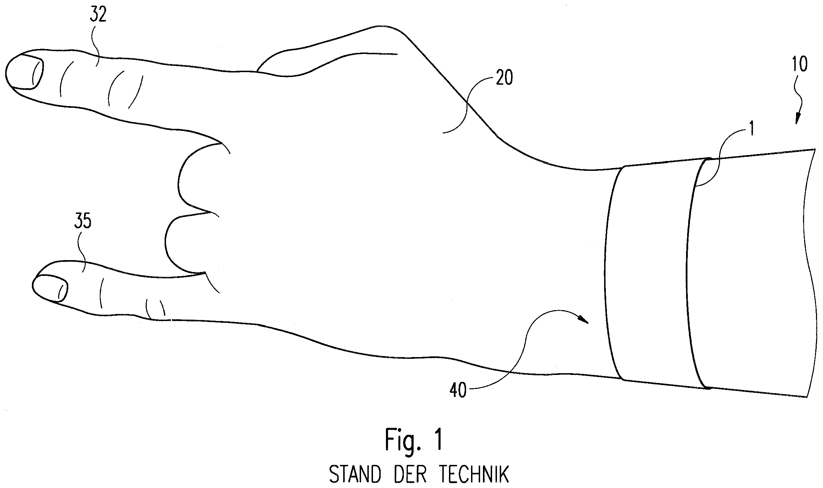

Auch ist aus dem Stand der Technik bekannt, Gesten eines Benutzers anhand einer Auswertung von Muskelimpulsen zu erkennen. Hierzu ist ein Armband bekannt, welches am Arm eines Benutzers angebracht und dazu ausgebildet ist, die Muskelimpulse des Benutzers auszulesen und dadurch die durch den Benutzer ausgeführten Gesten und Handbewegungen zu erkennen. Ein solches Armband 1 ist in der Figur 1 dargestellt. Das Armband 1 ist am Arm 10 eines Benutzers angebracht. In der Figur 1 ist die Hand 20 des Benutzers deutlich erkennbar. Der Benutzer führt eine Geste aus, bei der der Zeigefinger 32 und der kleine Finger 35 geöffnet werden, während die Handinnenfläche der Hand 20 nach unten zeigt. Dabei werden mittels des Armbandes 1 die beim Ausführen der vorhin genannten Geste in den Unterarmmuskeln 40 des Benutzers entstehenden Muskelimpulse erfasst.

Die Europäische Patentanmeldung EP 1 408 443 A1 offenbart ein System zum Ansteuern von Verbraucher einer Haushaltsleittechnik, mittels Muskelimpulsen eines Benutzers.

Ausgehend von dem bekannten Stand der Technik ist es nunmehr eine Aufgabe der Erfindung, ein System und ein Verfahren zum Ansteuern von Verbrauchern einer Haushaltsleittechnik, insbesondere von Leuchten, bereitzustellen, bei dem wenigstens ein Verbraucher der Hausleittechnik über solche durch wenigstens einen Benutzer ausgeführten Gesten in einer einfachen und intuitiven Weise angesteuert werden kann.

Diese Aufgabe wird durch den Gegenstand der unabhängigen Ansprüche gelöst. Die abhängigen Ansprüche bilden den zentralen Gedanken der Erfindung in besonders vorteilhafter Weise weiter.

Zur Lösung dieser Aufgabe wird erfindungsgemäß ein System zum Ansteuern von Verbrauchern einer Haushaltsleittechnik, insbesondere von Leuchten, mittels Muskelimpulsen wenigstens eines Benutzers, bereitgestellt. Dabei umfasst das erfindungsgemäße System wenigstens eine Muskelimpulserfassungseinheit zum Erfassen von elektrischen Muskelimpulsen, die beim Durchführen einer oder mehrerer im freien Raum erzeugten Gesten entstehen. Die Muskelimpulserfassungseinheit ist dazu ausgebildet, die Muskelimpulse in Form von Signalen, vorzugsweise von digitalen Signalen, die jeweils einer anderen Geste eindeutig zugeordneten sind, zu erfassen. Ferner weist die Muskelimpulserfassungseinheit eine Sendeeinheit zum Übermitteln der den Gesten eindeutig zugeordneten Signale auf. Weiterhin umfasst das erfindungsgemäße System wenigstens eine Steuereinheit, die eine Empfangseinheit zum Empfangen der von der Sendeeinheit übermittelten Signale und eine Zuordnungseinheit zum Zuordnen der empfangenen Signale zu in der Steuereinheit hinterlegten Steuerungsbefehlen bzw. Steuerbefehlen aufweist. Des Weiteren weist die erfindungsgemäße Steuereinheit eine Ansteuereinheit zum Ansteuern wenigstens eines Verbrauchers der Hausleittechnik auf Basis der den empfangenen Signalen zugeordneten Steuerungsbefehle auf.

Erfindungsgemäß wird ferner ein Verfahren zum Ansteuern von Verbrauchern einer Hausleittechnik, insbesondere von Leuchten, mittels Muskelimpulsen wenigstens eines Benutzers, bereitgestellt. Bei dem erfindungsgemäßen Verfahren werden elektrische Muskelimpulse wenigstens eines Benutzers, die beim Durchführen einer oder mehrerer im freien Raum erzeugten Gesten entstehen, mittels wenigstens einer Muskelimpulserfassungseinheit erfasst. Dabei werden die Muskelimpulse in Form von Signalen, vorzugsweise von digitalen Signalen, die jeweils einer anderen Geste eindeutig zugeordneten sind, erfasst. Ferner werden die den Gesten eindeutig zugeordneten Signale mittels einer Sendeeinheit der Muskelimpulserfassungseinheit übermittelt. Bei dem Verfahren werden dann die von der Sendeeinheit übermittelten Signale mittels einer Empfangseinheit wenigstens einer Steuereinheit empfangen. Weiterhin werden die empfangenen Signale zu in der Steuereinheit hinterlegten Steuerungsbefehlen mittels einer Zuordnungseinheit der Steuereinheit zugeordnet. Ferner wird wenigstens ein Verbraucher der Hausleittechnik auf Basis der den empfangenen Signalen zugeordneten Steuerungsbefehle mittels einer Ansteuereinheit der Steuereinheit angesteuert.

Die Erfindung ermöglicht sehr vorteilhaft, wenigstens einen Verbraucher einer Hausleittechnik, vorzugweise wenigstens eine Leuchte eines Beleuchtungssystems, über Gesten anzusteuern, indem Muskelströme in einem Arm wenigstens eines Benutzers direkt mittels wenigstens einer jeweils einem Benutzer zugeordneten erfindungsgemäßen Muskelimpulserfassungseinheit gemessen werden.

So wird es möglich, dass beispielsweise das von den ansteuerbaren Leuchten des Beleuchtungssystems stammende Licht über verschiedene durch wenigstens einen Benutzer ausgeführte Gesten, die solche durch den wenigstens einen Benutzer über einen weiten Bereich ausgeführte große und kleine Bewegungen umfassen, in einer intuitiven und spielerischen Weise anzusteuern.

Die Erfindung ermöglicht sehr vorteilhaft auch, dass die ansteuerbaren Verbraucher der Hausleittechnik oder die ansteuerbaren Leuchte des Beleuchtungssystems von mehreren Benutzern gleichzeitig angesteuert werden können. Dazu muss jeder der mehreren Benutzer mit einer erfindungsgemäßen Muskelimpulserfassungseinheit ausgerüstet sein, die die Muskelimpulse des zugeordneten Benutzers in Form von Signalen, die jeweils einer anderen Geste eindeutig zugeordnet sind, erfasst und die erfassten Signale an die erfindungsgemäße Steuereinheit sendet. Der Benutzer muss sich dabei jedoch nicht im Erfassungsbereich einer Kamera befinden, sondern kann sich frei im Raum bewegen. Zudem kann die Ansteuerung, anders als die Steuerung mittels Kameraerfassung, auch im Dunkeln durchgeführt werden. Eine Kamera ist nicht mehr notwendig und die damit verbundenen Nachteile können minimiert oder gar überwunden werden. Beispielsweise ist auch keine Kalibrierung des Systems mehr notwendig.

Vorzugsweise weist das erfindungsgemäße System wenigstens einen Verbraucher, insbesondere wenigstens eine Leuchte, auf, welcher mittels der Steuerungsbefehle ansteuerbar ist.

Auf diese Weise ist es möglich, dass über eine erfindungsgemäße Muskelimpulserfassungseinheit die Leuchten eines Beleuchtungssystems einer Hausleittechnik und weitere Verbraucher der Hausleittechnik, wie beispielsweise Computer, Fernseher, Radio, Jalousien und Klimageräte, auf einfache Weise und gleichzeitig angesteuert werden können.

Bevorzugt ist die wenigstens eine erfindungsgemäße Steuereinheit mit dem wenigstens einen Verbraucher mittels drahtgebundener Kommunikationsmittel, wie insbesondere DALI oder POWERLINE, oder drahtloser Kommunikationsmittel, wie insbesondere Bluetooth, zur Übertragung der Steuerungsbefehle von der Steuereinheit an den wenigstens einen Verbraucher verbunden. Ferner können die drahtgebundenen Kommunikationsmittel ein Bussystem umfassen.

Weiter bevorzugt weisen die Sendeeinheit der erfindungsgemäßen Muskelimpulserfassungseinheit und die Empfangseinheit der erfindungsgemäßen Steuereinheit drahtlose Kommunikationsmittel, vorzugsweise Bluetooth-Module, auf. Dabei sind die drahtlosen Kommunikationsmittel zur drahtlosen Signalübertragung vorgesehen. Ferner können die drahtlosen Kommunikationsmittel (der Empfangseinheit) der wenigstens einen Steuereinheit in den mittels der wenigstens einen Steuereinheit ansteuerbaren Verbraucher integriert sein; also bspw. als integriertes Drahtlosmodul als Teil des Verbrauchers vorgesehen sein.

Erfolgt die Übertragung der mittels der erfindungsgemäßen Muskelimpulserfassungseinheiten erfassten Signale drahtlos, so können die anzusteuernden Verbraucher der Hausleittechnik - bspw. die anzusteuernden Leuchten des Beleuchtungssystems - ortsunabhängig angesteuert werden, weil eine Muskelimpulserfassungseinheiten direkt an dem ihr zugeordneten Benutzer angebracht ist und jeder Benutzer sich nicht zu einem Bediengerät hin bewegen muss. Das heißt, dass die erfindungsgemäß realisierte Ansteuerung unabhängig von ortsgebundenen Bediengeräten, wie beispielsweise von ortsgebundenen Schaltern, Tasten, Touchdisplays oder von herkömmlichen Bediengeräten, bei denen eine Ansteuerung mittels solcher von ortsgebundenen Kameras aufgenommenen Bildern erfolgt, ausgeführt werden kann.

Ein Vorteil der Erfindung ist, dass sich ein Benutzer frei bewegen kann und sich nicht, wie beispielsweise bei einer konventionellen Gestenerfassung mittels Kameras, im Blickfeld der eingesetzten Kameras zum Aufnehmen von Bildern des Benutzers beim Ausführen der verschiedenen zum Ansteuern verwendeten Gesten befinden muss.

Ein weiterer Vorteil der Erfindung ist, dass die erfindungsgemäße Ansteuerung der Verbraucher eines Hausleitsystems oder beispielsweise der Leuchten eines Beleuchtungssystems auch im Dunkel möglich ist, das heißt, das bei der erfindungsgemäßen Ansteuerung kein Licht notwendig ist. Ferner ist es nicht notwendig eine Kalibrierung der erfindungsgemäßen Muskelimpulserfassungseinheiten durchzuführen, so wie diese bei der Verwendung mancher herkömmlichen Bediengeräte notwendig ist.

Bei einer bevorzuge Ausführungsform der Erfindung ist die erfindungsgemäße Steuereinheit dazu ausgebildet, wenigstens einen anzusteuernden Verbraucher mittels eines dem zuletzt empfangenen Signal zugeordneten Steuerungsbefehls anzusteuern. Vorzugsweise ist der Steuerungsbefehl dem innerhalb einer vorbestimmten Zeit zuletzt empfangenen Signal zugeordnet.

Auf diese Weise ist es möglich, dass der wenigstens eine Verbraucher der Hausleittechnik oder die wenigstens eine Leuchte des Beleuchtungssystems gleichzeitig durch mehrere Benutzer, die jeweils mit einer erfindungsgemäßen Muskelimpulserfassungseinheit ausgerüstet sind, ansteuerbar ist, da die wenigstens eine erfindungsgemäße Steuereinheit einzig das von einer erfindungsgemäßen Muskelimpulserfassungseinheit zuletzt empfangene Signal als gültiges Signal einstuft und nur einzig den diesem zuletzt empfangenen Signal zugeordneten Steuerungsbefehl zum Ansteuern des wenigstens einen Verbrauchers oder der wenigstens einen Leuchte ausführt.

Bei einer weiteren bevorzuge Ausführungsform der Erfindung ist die erfindungsgemäße Steuereinheit derart ausgebildet, dass mit einer Muskelimpulserfassungseinheit nur ein vordefinierter Verbraucher oder eine vordefinierte Gruppe von Verbrauchern ansteuerbar ist.

Auf diese Weise ist es möglich, dass ein Benutzer, der mit einer solchen erfindungsgemäßen Muskelimpulserfassungseinheit ausgerüstet ist, nur vordefinierte, ihm zugeordnete Verbraucher oder Leuchten ansteuern kann. Das ist beispielsweise bei der Ansteuerung der Leuchten eines im Bürobereich eingesetzten Beleuchtungssystems vorteilhaft, da jedem Angestellten eine Leuchte, beispielsweise die sich über seinem Arbeitsplatz befindliche Leuchte, zugeordnet werden kann. So kann dann jeder Angestellte gerade die ihm zugeordnete Leuchte über seine Muskelimpulserfassungseinheit ansteuern. Falls sich der Arbeitsplatz eines Angestellten ändert, so kann die sich über dem neuen Arbeitslatz befindliche Leuchte diesem Angestellten mittels der Software der Zuordnungseinheit der erfindungsgemäßen Steuereinheit neu zugeordnet werden.

Bei einer sehr vorteilhaften Ausführungsform der Erfindung ist die erfindungsgemäße Zuordnungseinheit ferner dazu ausgebildet, eine vordefinierte Gruppe von Signalen (d.h. entsprechend eine bestimmte Gruppe von Gesten, also ein bestimmter (vordefinierter) Gestensatz) zu in der Steuereinheit hinterlegten Auswahlbefehlen zuzuordnen. Weiterhin umfasst die erfindungsgemäße Steuereinheit eine Auswahleinheit, die ausgebildet ist, auf Basis der den empfangenen Signalen der vordefinierten Gruppe zugeordneten Auswahlbefehlen einen oder eine Gruppe von anzusteuernden Verbrauchern auszuwählen, um einzig die ausgewählten Verbraucher mittels der Steuerungsbefehle anzusteuern.

Auf diese Weise kann ein Benutzer, der mit einer erfindungsgemäßen Muskelimpulserfassungseinheit ausgerüstet ist, zuerst diejenigen Verbraucher oder Leuchten durch Ausführen von vordefinierten Gesten auswählen, die er ansteuern möchte. Unmittelbar danach kann dann derselbe Benutzer die ausgewählten Verbraucher oder Leuchten durch Ausführen von anderen vordefinierten Gesten wie gewünscht ansteuern. In dem vorgenannten Fall sind den Steuerungsbefehlen andere Signale bzw. Gesten oder Gestensätze zugeordnet als den Auswahlbefehlen. Es ist jedoch auch denkbar, dass einer Geste bzw. dem entsprechenden empfangenen Signal zugleich wenigstens ein Auswahlbefehl sowie wenigstens ein Steuerungsbefehl zugeordnet ist/wird; bspw. wähle Gruppe X (bspw. mehrere Leuchten eines Beleuchtungssystems) und führe Funktion Y (bspw. Dimmen oder An-/Ausschalten) aus.

Bei einer weiteren sehr vorteilhaften Ausführungsform der Erfindung weist die erfindungsgemäße Steuereinheit eine Speichereinheit auf. Ferner ist die erfindungsgemäße Steuereinheit dazu ausgebildet, ein empfangenes Signal einem Auswahlbefehl und/oder einem Steuerungsbefehl zuzuordnen oder einem Auswahlbefehl und/oder einem Steuerungsbefehl ein empfangenes Signal zuzuordnen und diese Zuordnung in der Speichereinheit zu hinterlegen.

Auf diese Weise können Auswahlbefehle und Steuerungsbefehle in einer sehr einfachen Weise über unterschiedliche Gesten ausgelöst werden.

Vorzugsweise umfassen die im freien Raum erzeugten Gesten, über die die Steuerungsbefehle und/oder Auswahlbefehle ausgelöst werden, ein Wischen mit einer Hand entlang einer ersten Richtung und/oder ein Wischen mit einer Hand entlang einer der ersten Richtung entgegengesetzten Richtung und/oder ein Öffnen einer Faust und/oder ein Schließen einer Faust und/oder ein Bewegen eines Armes entlang einer gegenüber der ersten Richtung senkrecht verlaufenden zweiten Richtung und/oder ein Bewegen eines Armes entlang einer der zweiten Richtung entgegengesetzten Richtung, ein Rotieren eines Armes in einer ersten Rotationsrichtung und/oder ein Rotieren eines Armes in einer der ersten Rotationsrichtung entgegengesetzten Rotationrichtung und/oder ein Fingerschnippen und/oder ein Öffnen einzelner Finger einer Hand und/oder ein Schließen einzelner Finger einer Hand und/oder ein Öffnen einer ersten Anzahl von Fingern einer Hand, deren Handinnenfläche in die erste Richtung zeigt, und/oder ein Öffnen einer zweiten Anzahl von Fingern einer Hand, deren Handinnenfläche in die der zweiten Richtung entgegengesetzten Richtung zeigt sowie alle weiteren denkbaren Gesten, welche mit einer Muskelimpulserfassungseinheit erfasst werden können und beispielsweise auch an anderen Körperteilen (bspw. Beinen, Kopf, etc.) vorgesehen sind.

Vorzugsweise umfassen die mittels der erfindungsgemäßen Ansteuereinheit durchführbaren Steuerungsbefehle das Ein- und Ausschalten wenigstens einer jeweils als ein Verbraucher dienenden Leuchte und/oder die Einstellung einer Farbtemperatur, Helligkeit und/oder weiterer Parameter wenigstens einer jeweils als ein Verbraucher dienenden Leuchte.

Weiter bevorzugt umfassen die im freien Raum erzeugten Gesten, über die die Auswahlbefehle ausgelöst werden, ein Öffnen einer ersten Anzahl von Fingern einer Hand, deren Handinnenfläche in die erste Richtung zeigt, und/oder ein Öffnen einer zweiten Anzahl von Fingern einer Hand, deren Handinnenfläche in die der zweiten Richtung entgegengesetzten Richtung zeigt.

Bei einer sehr vorteilhaften Ausführungsform der Erfindung ist eine erfindungsgemäße Steuereinheit je einem Verbraucher, einer Gruppe von Verbrauchern oder allen Verbrauchern des Systems zugeordnet. Dabei ist die erfindungsgemäße Steuereinheit dazu ausgebildet, den ihr zugeordneten Verbraucher oder die ihr zugeordneten Verbraucher wahlweise anzusteuern. Die erfindungsgemäße Steuereinheit ist vorzugsweise als Zentralsteuereinheit (über die alle oder eine (vor-)definierte Gruppe von Verbrauchern wahlweise ansteuerbar ist), als separate Steuereinheit (über die wahlweise mehrere Verbraucher ansteuerbar sind) oder als Teil eines ihr zugeordneten Verbrauchers (über das der/die entsprechende(n) Verbraucher ansteuerbar ist/sind) ausgebildet.

Bei einer sehr vorteilhaften Ausführungsform der Erfindung weist die erfindungsgemäße Muskelimpulserfassungseinheit Sensoren zur Erfassung von Muskelimpulsen auf. Diese Sensoren können neben Muskelimpulserfassungssensoren auch bspw. Gyrosensoren, Beschleunigungssensore und dergleichen aufweisen, die zur umfassenden Erfassung einer Geste genutzt werden können. Insbesondere kann die Muskelimpulserfassungseinheit als Armband oder dergleichen ausgebildet sein, welches die vorgenannten Sensoren aufweist und bspw. an dem Arm des Benutzers zur Erfassung der Muskelimpulse angebracht werden kann.

Bei dieser Ausführungsform der Erfindung können die Sensoren eines am Arm eines Benutzers angebrachten Armbandes die Muskelimpulse des Benutzers messen. Das Armband sendet dann die in Form von jeweils einer anderen Geste zugeordneten Signalen erfassten Muskelimpulse vorzugsweise über eine drahtlose Kommunikation, beispielsweise über Bluetooth, an die erfindungsgemäße Steuereinheit weiter. Dabei können durch das Armband verschiedenste Gesten erkannt werden.

Mithilfe einer im erfindungsgemäßen System enthaltenen Software kann je einer anderen Geste je ein anderer Auswahl- oder Steuerungsbefehl zugeordnet werden. Nachdem die gewünschte Geste ausgeführt wurde, wird der ausgeführten Geste der entsprechende Auswahl- oder Steuerungsbefehl bevorzugt mittels derselben Software zugeordnet. Die entsprechende Zuordnung wird daraufhin weiter bevorzugt durch die Software oder das Armband gespeichert. Umgekehrt ist es auch denkbar, dass einem gespeicherten Auswahl- oder Steuerungsbefehl eine Geste zugeordnet wird. Hierzu wird ein gewünschter Auswahl- oder Steuerungsbefehl ausgewählt und die ihm zugeordnete Geste ausgeführt. Das entsprechend empfangene Signal wird dann dem Auswahl- oder Steuerungsbefehl zugeordnet und hinterlegt.

Beispielsweise kann ein erfindungsgemäßes System im Büro- beziehungsweise im Großraumbürobereich eingesetzt werden. So können die Leuchten eines in diesem Bereich verwendeten Beleuchtungssystems von mehreren Angestellten gleichzeitig angesteuert werden. Vorzugsweise kann jeder Angestellte direkt von seinem Arbeitsplatz aus eine sich an seinem Arbeitsplatz befindliche Leuchte ansteuern. So kann jedem Angestellten die entsprechende Leuchte zugeordnet werden, die er durch einfache Gesten ansteuern kann. Auf diese Weise muss kein Angestellter aufstehen und zu einem Bediengerät gehen, um dort umständlich die richtige Leuchte auszusuchen. Die Auswahl- oder Steuerungsbefehle werden über einen Router an eine Zentralsteuereinheit, weitergeleitet, die die einzelnen Leuchten ansteuert. Eine solche Zentralsteuereinheit kann hier in Form eines Lichtmanagementsystems ausgebildet sein. Bevorzugt kann das von jeder Leuchte stammende Licht mit einem Verschließen einer Faust runtergedimmt werden. Weiter bevorzugt kann durch einen Wisch jede Leuchte ausgeschaltet werden. Durch den Einsatz des erfindungsgemäßen Systems im Büro- beziehungsweise im Großraumbürobereich ergeben sich hohe Energieeinsparungsmöglichkeiten, da heute meist alle Leuchten eines Beleuchtungssystems eingeschaltet bleiben, bis der letzte Angestellte den entsprechenden Büroraum verlässt.

Weiterhin kann ein erfindungsgemäßes System im privaten Bereich eingesetzt werden. So kann in jeder Leuchte eines Beleuchtungssystems einer Hausleittechnik eine erfindungsgemäße Steuereinheit, die vorzugsweise ein Drahtlosmodul umfasst, integriert werden. Die Leuchten können dann drahtlos über die integrierten Steuereinheiten jeweils direkt angesteuert werden. Mittels des Armbandes kann in einer sehr einfachen Weise beispielsweise am Abend eine wärmere Farbtemperatur für eine entspannte Atmosphäre eingestellt werden. Optional können über das Armband die Leuchten des Beleuchtungssystems und gleichzeitig weitere Verbraucher beziehungsweise Geräte wie beispielsweise Computer und/oder Fernseher und/oder Radio und/oder Jalousien und/oder Klimageräte angesteuert werden. In diesem Fall können alle Verbraucher einer Hausleittechnik mittels eines einzelnen Armbandes angesteuert beziehungsweise bedient werden, wodurch die Vielzahl unterschiedlicher Bediengeräte beziehungsweise Fernbedienungen, die üblicherweise zum Bedienen beziehungsweise Ansteuern der verschiedener Verbraucher beziehungsweise Geräte einer Hausleittechnik notwendig sind, hinfällig werden.

Auch kann ein erfindungsgemäßes System im Präsentations- und Verkaufsbereich eingesetzt werden. Ein Angestellter kann in einer sehr einfachen Weise mittels eines erfindungsgemäßen Armbandes spezifische Leuchten beziehungsweise Strahler eines in diesem Bereich eingesetzten erfindungsgemäßen Beleuchtungssystems über einfache Gesten beziehungsweise Arm- und Handbewegungen ansteuern, um Waren gezielt in Szene zu setzen. Dadurch, dass das erfindungsgemäße Systen in einer sehr intuitiven und dadurch auch sehr einfachen Weise bedient werden kann, können die Leuchten beziehungsweise die Strahler eines solchen Beleuchtungssystems auch von ungelernten Personal optimal ansteuert werden.

Ferner kann ein erfindungsgemäßes System auch im Hotel- und Wellnessbereich eingesetzt werden. Heutzutage ist es relativ umständlich die Leuchten eines herkömmlichen in einem Hotelzimmer eingesetzten Beleuchtungssystems anzusteuern, da sich die zahlreichen Schalter für die verschiedenen Leuchten eines solchen herkömmlichen Beleuchtungssystems oft verteilt in dem entsprechenden Raum befinden. Dabei ist die Zuordnung der Schalter zu den genannten Leuchten unbekannt. Ein Benutzer beziehungsweise ein Hotelgast kann nur durch ein mühsames Versuch-Irrtum-Verfahren herausfinden, welcher Schalter der richtige Schalter ist. Das Vorhandensein von zahlreichen, in dem Hotelzimmer verteilten Schaltern bringt Komforteinbüßen mit sich, wie beispielsweise in dem Fall, in dem vergessen worden ist, das Licht im Badezimmer auszuschalten. In diesem Fall muss der Hotelgast wieder aufstehen, um das Licht im Badezimmer auszuschalten. Bei der Verwendung eines erfindungsgemäßen Systems braucht sich der Hotelgast nur eine kurze Bedienungsanleitung anzueignen, um spielerisch das von den Leuchten eines erfindungsgemäßen Beleuchtungssystems stammende Licht über Gesten beziehungsweise Hand- und Armbewegungen anzusteuern. Neben dem Spaßfaktor bietet die erfindungsgemäße Ansteuerung der Leuchten auch einen erhöhten Komfort, da das Bediengerät, das vorzugsweise als Armband ausgebildet ist, immer direkt am Benutzer beziehungsweise am Hotelgast ist.

Des Weiteren kann ein erfindungsgemäßes System auch im Gesundheits- und Pflegebereich eingesetzt werden. Durch die Verwendung eines erfindungsgemäßen Armbandes wird pflegebedürftigen Personen und Personen, die in ihrer Bewegungsfähigkeit eingeschränkt sind, ermöglicht, über Gesten beziehungsweise Arm- und Handbewegungen das von den Leuchten eines erfindungsgemäßen Beleuchtungssystems stammende Licht anzusteuern. Oft wäre es diesen Personen sonst nicht mehr möglich, eigenständig das Licht anzusteuern.

Alternativ kann ein erfindungsgemäßes System im Bereich der Fassaden- und Architekturbeleuchtung eingesetzt werden. Die Verwendung eines erfindungsgemäßen Armbandes zum Ansteuern der Leuchten eines erfindungsgemäßen Beleuchtungssystems, das zum Beleuchten einer Fassade vorgesehen ist, bietet einen künstlerischen, spektakulären Aspekt, weil der Benutzer, an dessen Arm ein erfindungsgemäßes Armband angebracht ist, ähnlich einem Dirigenten, die Leuchten des erfindungsgemäßen Beleuchtungssystems zum Beleuchten der Fassade über Gesten beziehungsweise Hand- und Armbewegungen ansteuern kann.

Einige Ausführungsbeispiele der Erfindung werden nachfolgend anhand der beigefügten Zeichnungen näher erläutert. Gleiche Bezugszeichen werden für die gleichen Komponenten verwendet. Darin zeigen:
- Figur 1: eine an dem Arm eines Benutzer angebrachte und aus dem Stand der Technik bekannte Muskelimpulserfassungseinheit (hier bspw. Armband) zum Erfassen der beim Ausführen einer klassischen Geste entstehenden Muskelimpulse;
- Figur 2: ein Blockschaltbild eines erfindungsgemäßen Systems nach einer ersten Ausführungsform der Erfindung;
- Figur 3: eine an dem Arm eines Benutzer angebrachte Muskelimpulserfassungseinheit des erfindungsgemäßen Systems nach der ersten Ausführungsform der Erfindung, die beim Erfassen der beim Ausführen einer ersten Geste entstehenden Muskelimpulse dargestellt ist;
- Figur 4: die in der Figur 3 dargestellte und an dem Arm desselben Benutzers angebrachte Muskelimpulserfassungseinheit des erfindungsgemäßen Systems nach der ersten Ausführungsform der Erfindung, die beim Erfassen der beim Ausführen einer zweiten Geste entstehenden Muskelimpulse dargestellt ist;
- Figur 5: ein Blockschaltbild eines erfindungsgemäßen Systems nach einer zweiten Ausführungsform der Erfindung; und
- Figur6: ein Blockschaltbild eines erfindungsgemäßen Systems nach einer dritten Ausführungsform der Erfindung.

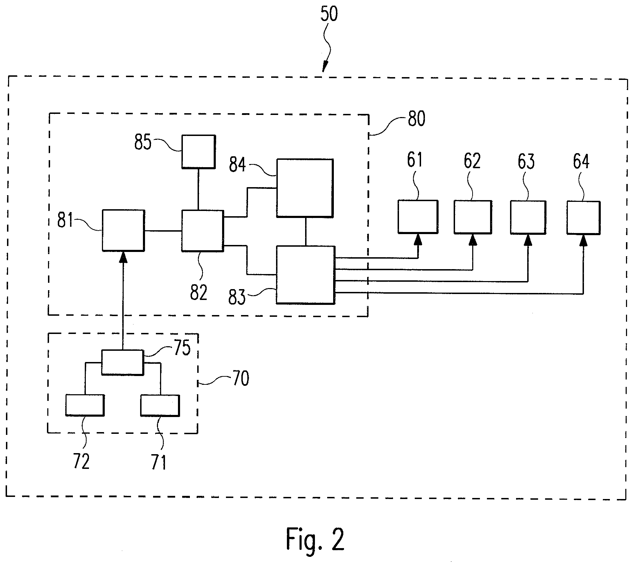

In der Figur 2 ist ein Blockschaltbild eines erfindungsgemäßen Systems 50 nach einer ersten Ausführungsform der Erfindung dargestellt. Das System 50 umfasst mehrere Verbraucher 61, 62, 63, 64 einer Haushaltsleittechnik. Dabei ist wenigstens einer der Verbraucher 61, 62, 63, 64, insbesondere alle Verbraucher 61, 62, 63, 64, über die Muskelimpulse wenigstens eines Benutzers (nicht dargestellt) ansteuerbar. Beispielsweise können die Verbraucher 61, 62 jeweils als eine Leuchte eines Beleuchtungssystems ausgebildet sein. Ferner können die Verbraucher 63, 64 jeweils als ein weiterer Verbraucher der Hausleittechnik ausgebildet sein. Solche weiteren Verbraucher 63, 64 der Hausleittechnik können beispielsweise Computer und/oder Fernseher und/oder Radios und/oder Jalousien und/oder Klimageräte sein. Auch können die Verbraucher 61, 62, 63, 64 jeweils als eine Leuchte eines Beleuchtungssystems ausgebildet sein.

Das System 50 weist ferner wenigstens eine Muskelimpulserfassungseinheit 70 auf, die vorzugsweise mehrere Sensoren 71, 72 und eine Sendeeinheit 75 umfasst. Das System 50 kann folglich auch mehrere Muskelimpulserfassungseinheiten 70 aufweisen, von denen zur Vereinfachung der Darstellung nur eine Muskelimpulserfassungseinheit 70 dargestellt ist. Vorzugsweise sind dann mehrere Benutzer jeweils mit einer Muskelimpulserfassungseinheit 70 ausgerüstet bzw. mehrere Muskelimpulserfassungseinheiten 70 sind für mehrere Benutzer bereitgestellt. Das System kann um beliebig viele Muskelimpulserfassungseinheiten 70 erweitert werden.

Insbesondere die Sensoren 71, 72 einer Muskelimpulserfassungseinheit 70 sind dazu ausgebildet, jeweils elektrische Muskelimpulse eines entsprechenden Benutzers, die beim Durchführen einer oder mehrerer im freien Raum erzeugten Gesten entstehen, zu erfassen. Dabei erfassen die Sensoren 71, 72 die genannten Muskelimpulse in Form von Signalen, die einer Geste eindeutig zugeordnet sind. Diese Signale sind vorzugsweise digitale Signale. Ferner ist die Sendeeinheit 75 einer Muskelimpulserfassungseinheit 70 dazu ausgebildet, die den Gesten eindeutig zugeordneten Signale, die vorzugsweise durch die genannten Sensoren 71, 72 erfasst wurden, zu übermitteln. Ergänzend ist denkbar, dass auch Sensoren zum Erfassen von Bewegungen und dergleichen vorgesehen sind, um eine Geste in seiner Gesamtheit - ggf. unter Einbeziehung von Bewegung/Beschleunigung bspw. mittels Gyrosensoren oder Beschleunigungssensoren - zu erfassen. Jede Muskelimpulserfassungseinheit 70 kann in Form eines Armbandes ausgebildet sein, das an dem Arm eines entsprechenden Benutzers anbringbar ist.

Das System 50 weist weiterhin eine zentrale Steuereinheit 80 auf, die eine Empfangseinheit 81, eine Zuordnungseinheit 82 und eine Ansteuereinheit 83 umfasst. Dabei ist die Empfangseinheit 81 dazu ausgebildet, die von der Sendeeinheit 75 der wenigstens einen Muskelimpulserfassungseinheit 70 übermittelten Signale zu empfangen. Hierzu weisen die Sendeeinheit 75 der Muskelimpulserfassungseinheit 70 und die Empfangseinheit 81 der Steuereinheit 80 vorzugsweise drahtlose Kommunikationsmittel auf, insbesondere Bluetooth-Module, zur drahtlosen Signalübertragung. Ferner ist die Zuordnungseinheit 82 dazu ausgebildet, die empfangenen Signale zu in der Steuereinheit 80 hinterlegten Steuerungsbefehlen zuzuordnen. Weiterhin ist die Ansteuereinheit 83 dazu ausgebildet, wenigstens einen der Verbraucher 61, 62, 63, 64 oder alle Verbraucher 61, 62, 63, 64 der Hausleittechnik auf Basis der den empfangenen Signalen zugeordneten Steuerungsbefehle jeweils anzusteuern.

Solche Steuerungsbefehle können beispielsweise das Ein- und Ausschalten und/oder die Einstellung einer Farbtemperatur, Helligkeit und/oder weiterer Parameter wenigstens einer jeweils als ein Verbraucher 61, 62, 63, 64 dienenden Leuchte umfassen. Auch ist bei einer Jalousie als Verbraucher das Öffnen oder Schließen derselben als Steuerungsbefehl denkbar. Die Erfindung ist jedoch nicht auf die vorgenannten Steuerungsbefehle beschränkt, sondern umfasst alle denkbaren Steuerungsbefehle; insbesondere für entsprechende Verbraucher einer Hausleittechnik.

Vorzugsweise ist die Zuordnungseinheit 82 ferner dazu ausgebildet, eine vordefinierte Gruppe von Signalen zu in der Steuereinheit 80 hinterlegten Auswahlbefehlen zuzuordnen. Dabei weist die Steuereinheit 80 bevorzugt eine Auswahleinheit 84 auf, die dazu ausgebildet ist, auf Basis der den empfangenen Signalen der vordefinierten Gruppe zugeordneten Auswahlbefehle jeweils einen der Verbraucher 61, 62, 63, 64 oder eine Gruppe von Verbrauchern aus den Verbrauchern 61, 62, 63, 64 auszuwählen. Vorzugsweise ist die Ansteuereinheit 83 dazu ausgebildet, einzig die ausgewählten Verbraucher mittels der Steuerungsbefehle anzusteuern. Beispielsweise umfasst eine solche Gruppe die Verbraucher 61, 62 und 63 und eine weitere Gruppe die Verbraucher 63 und 64.

Das bedeutet, dass die mittels der Sendeeinheit 75 einer Muskelimpulserfassungseinheit 70 gesendeten Signale, die mittels der Zuordnungseinheit 82 zu den zu verwendenden Steuerungsbefehlen zugeordnet werden, vorzugsweise anderen Gesten bzw. einem anderen Gestensatz eindeutig zugeordnet sind, als die mittels der Sendeeinheit 75 einer Muskelimpulserfassungseinheit 70 gesendeten Signale, die mittels der Zuordnungseinheit 82 zu den zu verwendenden Auswahlbefehlen zugeordnet werden. Das bedeutet ferner, dass die Steuerungsbefehle von anderen Gesten ausgelöst werden als die Auswahlbefehle. Es ist jedoch auch denkbar, dass mit einer (vordefinierten) Geste gleichzeitig ein Auswahlbefehl ausgeführt und für die so ausgewählten Verbraucher sodann ein Steuerungsbefehl ausgegeben wird; bspw. "wähle Verbraucher 61 und 62 und schalte diese ein". Auch können mit einer Geste unterschiedlichen Verbrauchern unterschiedliche Befehle zugedacht werden. So kann bspw. bei Ausführen einer vordefinierten Geste ein Verbraucher 61 (bspw. Leuchte) angeschaltet und ein anderer Verbraucher 64 (bspw. Jalousie) geschlossen werden.

In dem Fall, in dem jeder Verbraucher 61, 62, 63, 64 als eine Leuchte eines Beleuchtungssystems ausgebildet ist, kann ein Wischen mit einer Hand nach Rechts einen ersten Steuerungsbefehl auslösen, der ein Einschalten aller jeweils als eine Leuchte ausgebildeten Verbraucher 61, 62, 63, 64 umfasst, ein Wischen mit einer Hand nach Links kann einen zweiten Steuerungsbefehl auslösen, der ein Ausschalten aller jeweils als eine Leuchte ausgebildeten Verbraucher 61, 62, 63, 64 umfasst, ein Heben eines Armes kann einen dritten Steuerungsbefehl auslösen, der das Hochdimmen aller jeweils als eine Leuchte ausgebildeten Verbraucher 61, 62, 63, 64 umfasst, und ein Senken eines Armes kann einen vierten Steuerungsbefehl auslösen, der das Runterdimmen aller jeweils als eine Leuchte ausgebildeten Verbraucher 61, 62, 63, 64 umfasst.

In demselben Fall kann ferner ein Öffnen des Daumens, des Zeigefingers und des Mittelfingers einer Hand, deren Handinnenfläche nach oben zeigt, einen ersten Auswahlbefehl auslösen, der das Auswählen der ersten drei jeweils als Leuchte ausgebildeten Verbraucher 61, 62, 63 umfasst, ein Öffnen des Zeigefingers und des Mittelfingers einer Hand, deren Handinnenfläche nach unten zeigt, kann einen zweiten Auswahlbefehl auslösen, der das Auswählen der letzten zwei jeweils als Leuchte ausgebildeten Verbraucher 62, 63 umfasst, ein Öffnen einer Faust einer Hand kann einen fünften Steuerungsbefehl auslösen, der das Hochdimmen der ausgewählten und jeweils als Leuchte ausgebildeten Verbraucher umfasst, und ein Schließen einer Faust einer Hand kann einen sechsten Steuerungsbefehl auslösen, der das Runterdimmen der ausgewählten und jeweils als Leuchte ausgebildeten Verbraucher umfasst. Diese Auswahl kann dann gespeichert werden (bspw. in einer im Folgenden noch beschriebenen Speichereinheit 85) und anschließend kann für die zuletzt ausgewählten Verbraucher eine Steuerungsbefehl-auslösende Geste ausgeführt werden. Alternativ ist es auch denkbar, die Gesten zum Ausführen der Steuerungsbefehle (bspw. Heben Senken oder Schwenken des Armes) mit einer entsprechenden Auswahlgeste (bspw. entsprechende Finger ausgestreckt) zu kombinieren, um definiert ausgewählte Verbraucher gleichzeitig anzusteuern.

Vorzugsweise umfasst die Steuereinheit 80 des Systems nach der ersten Ausführungsform der Erfindung eine Speichereinheit 85. Dabei ist die Steuereinheit bevorzugt dazu ausgebildet, ein empfangenes Signal einem Auswahlbefehl oder einem Steuerungsbefehls zuzuordnen oder einem Auswahlbefehl oder einem Steuerungsbefehl ein empfangenes Signal zuzuordnen und diese Zuordnung in der Speichereinheit 85 zu hinterlegen.

In dem Fall, in dem das System 50 nach der ersten Ausführungsform der Erfindung mehrere Muskelimpulserfassungseinheiten 70 umfasst, können die anzusteuernden Verbraucher der Verbraucher 61, 62, 63, 64 bevorzugt von mehreren Benutzern, die jeweils mit einer Muskelimpulserfassungseinheit 70 ausgerüstet sind, gleichzeitig angesteuert werden. Dazu ist die Steuereinheit 80 vorzugsweise dazu ausgebildet, wenigstens einen anzusteuernden Verbraucher der Verbraucher 61, 62, 63, 64 mittels eines dem - vorzugsweise innerhalb einer vorbestimmten Zeit - zuletzt empfangenen Signal zugeordneten Steuerungsbefehls anzusteuern.

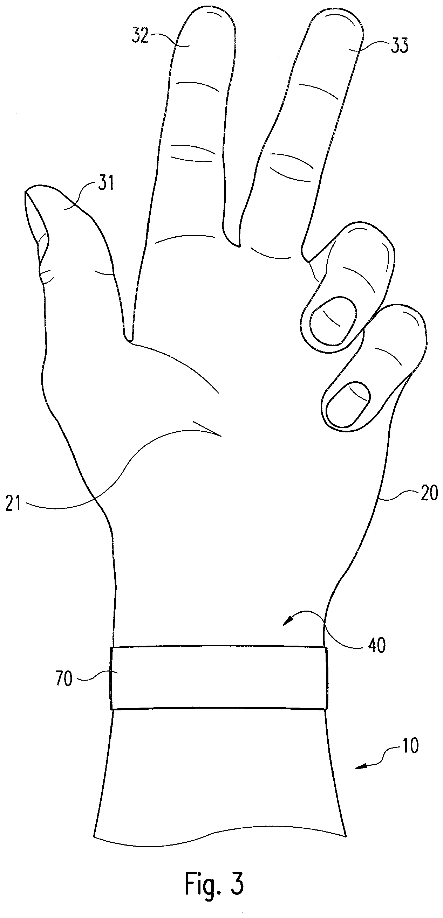

Figur 3 zeigt beispielhaft eine als ein Armband ausgebildete erfindungsgemäße Muskelimpulserfassungseinheit 70, die in dem System 50 nach der ersten Ausführungsform der Erfindung einsetzbar und am Arm 10 eines Benutzers angebracht ist. In der Figur 3 führt der Benutzer eine erste Geste aus, bei der der Daumen 31, der Zeigefinger 32 und der Mittelfinger 33 einer Hand 20 geöffnet sind/werden, während die Handinnenfläche 21 der Hand 20 nach oben zeigt. Durch diese Geste kann beispielsweise ein erster Auswahlbefehl ausgelöst werden, der ein Auswählen der ersten drei jeweils als Leuchte ausgebildeten Verbraucher 61, 62, 63 auslöst. Dabei werden mittels der als ein Armband ausgebildeten Muskelimpulserfassungseinheit 70 die beim Ausführen der ersten Geste in den Unterarmmuskeln 40 des Benutzers entstehenden Muskelimpulse erfasst und mittels der Sendeeinheit 75 als (digitale) Signale der Steuereinheit 80 bereitgestellt.

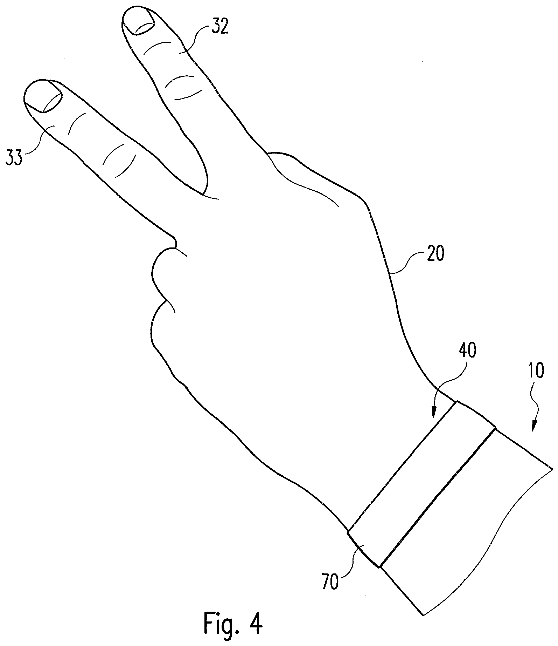

Figur 4 zeigt dieselbe als ein Armband ausgebildete erfindungsgemäße Muskelimpulserfassungseinheit 70 aus der Figur 3, die in dem System 50 nach der ersten Ausführungsform der Erfindung einsetzbar und am Arm 10 desselben Benutzers angebracht ist. In der Figur 4 führt der Benutzer eine zweite Geste aus, bei der der Zeigefinger 32 und der Mittelfinger 33 einer Hand 20 geöffnet werden, während die Handinnenfläche der Hand 20 nach unten zeigt. Durch diese Geste kann beispielsweise ein zweiter Auswahlbefehl ausgelöst werden, der ein Auswählen der letzten zwei jeweils als Leuchte ausgebildeten Verbraucher 63, 64 auslöst. Dabei werden mittels der als ein Armband ausgebildeten Muskelimpulserfassungseinheit 70 die beim Ausführen der zweiten Geste in den Unterarmmuskeln 40 des Benutzers entstehenden Muskelimpulse erfasst.

Es sei darauf hingewiesen, dass die Muskelimpulserfassungseinheit 70 grundsätzlich auch anders ausgebildet sein kann und an einem oder mehreren anderen Körperteil(en) des Benutzers befestigt werden kann, solange mit ihr (vor-)definierte Gesten erfassbar und als Signale übertragbar sind.

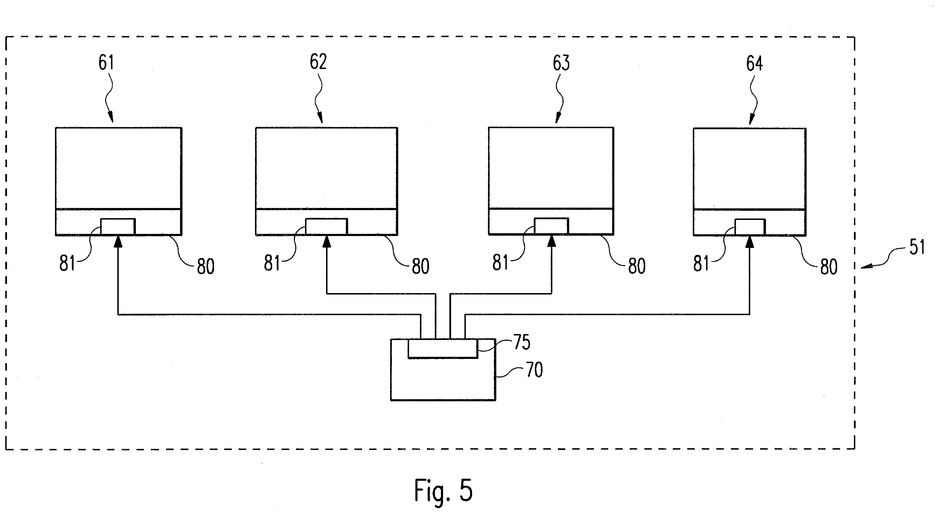

Figur 5 zeigt ein Blockschaltbild eines erfindungsgemäßen Systems 51 nach einer zweiten Ausführungsform der Erfindung. Das erfindungsgemäße System 51 nach der zweiten Ausführungsform der Erfindung unterscheidet sich alleine darin von dem erfindungsgemäßen System 50 nach der ersten Ausführungsform der Erfindung, dass das erfindungsgemäße System 51 nach der zweiten Ausführungsform der Erfindung mehrere Steuereinheiten 80 umfasst, die jeweils in einem zugeordneten Verbraucher der Verbraucher 61, 62, 63, 64 integriert sind und jeweils dazu ausgebildet sind, einzig den jeweils zugeordneten Verbraucher der Verbraucher 61, 62, 63, 64 ansteuern.

Jede Steuereinheit 80 des erfindungsgemäßen Systems 51 nach der zweiten Ausführungsform der Erfindung weist dieselbe Struktur auf wie die Steuereinheit 80 des erfindungsgemäßen Systems 50 nach der ersten Ausführungsform der Erfindung. Jede Muskelimpulserfassungseinheit 70 des erfindungsgemäßen Systems 51 nach der zweiten Ausführungsform der Erfindung weist dieselbe Struktur auf wie die dargestellte und zuvor beschriebene Muskelimpulserfassungseinheit 70 des erfindungsgemäßen Systems 50 nach der ersten Ausführungsform der Erfindung.

Genauso wie das erfindungsgemäße System 50 nach der ersten Ausführungsform der Erfindung umfasst das erfindungsgemäße System 51 nach der zweiten Ausführungsform der Erfindung eine oder mehrere Muskelimpulserfassungseinheiten 70. Zur Vereinfachung der Darstellung wird auch in der Figur 5 nur eine Muskelimpulserfassungseinheit 70 dargestellt.

Auch bei der zweiten Ausführungsform der Erfindung weist jede Steuereinheit 80 eine Empfangseinheit 81 zum Empfangen von mittels der Sendeeinheit 75 der einzelnen Muskelimpulserfassungseinheiten 70 oder mittels der Sendeeinheiten 75 einer jeden der mehreren Muskelimpulserfassungseinheiten 70 gesendeten Signale. Jede Steuereinheit 80 ist weiter dazu ausgebildet, die empfangenen Signale - genauso wie die Steuereinheit 80 des erfindungsgemäßen Systems 50 nach der ersten Ausführungsform der Erfindung - weiter zu verarbeiten beziehungsweise zu verwenden. Dabei ist jede Steuereinheit 80 dazu ausgebildet, einzig denjenigen Verbraucher der Verbraucher 61, 62, 63, 64 anzusteuern, in dem sie jeweils integriert ist.

Um die Übertragung der Signale von der dargestellten Muskelimpulserfassungseinheit 70 zu den Steuereinheiten 80 zu verdeutlichen, sind in der Figur 5 nur die Sendeeinheit 75 der eingezeichneten Muskelimpulserfassungseinheit 70 und die Empfangseinheiten 81 der Steuereinheiten 80 dargestellt.

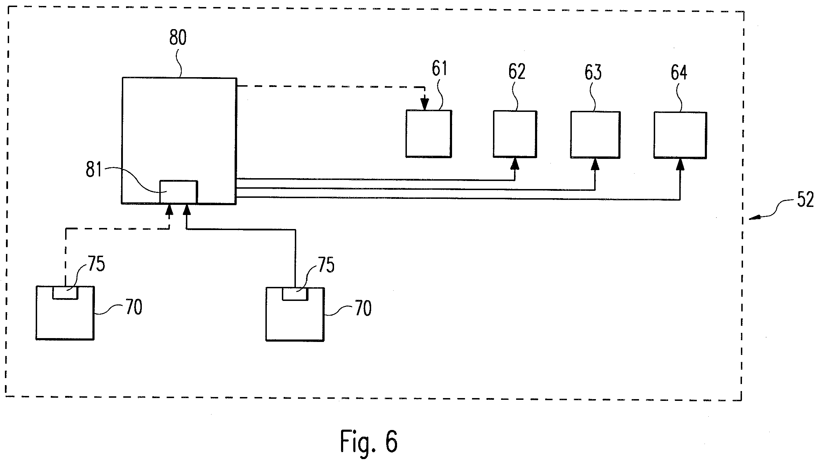

Figur 6 zeigt ein Blockschaltbild eines erfindungsgemäßen Systems 52 nach einer dritten Ausführungsform der Erfindung. Figur 6 zeigt hierzu beispielhaft eine Steuereinheit 80 und zwei Muskelimpulserfassungseinheiten 70. Die Steuereinheit 80 des erfindungsgemäßen Systems 52 nach der dritten Ausführungsform der Erfindung weist dieselbe Struktur auf wie die Steuereinheit 50 des erfindungsgemäßen Systems nach der ersten Ausführungsform der Erfindung. Jede Muskelimpulserfassungseinheit 70 des erfindungsgemäßen Systems 52 nach der dritten Ausführungsform der Erfindung weist dieselbe Struktur auf wie die dargestellte und zuvor beschriebene Muskelimpulserfassungseinheit 70 des erfindungsgemäßen System 50 nach der ersten Ausführungsform der Erfindung auf.

Das erfindungsgemäße System 52 nach der dritten Ausführungsform der Erfindung unterscheidet sich alleine darin von dem erfindungsgemäßen System 50 nach der ersten Ausführungsform der Erfindung, dass die Steuereinheit 80 des erfindungsgemäßen Systems 52 nach der dritten Ausführungsform der Erfindung eine weitere Funktion hat, das heißt, dass die Steuereinheit 80 des erfindungsgemäßen Systems 52 nach der dritten Ausführungsform der Erfindung zusätzlich derart ausgebildet ist, dass beispielsweise mit einer der zwei Muskelimpulserfassungseinheiten 70 nur ein vordefinierter Verbraucher, wie beispielsweise der Verbraucher 61, ansteuerbar beziehungsweise auswählbar und ansteuerbar ist und mit der anderen der zwei Muskelimpulserfassungseinheiten 70 ein oder eine vordefinierte Gruppe von Verbraucher(n), die beispielsweise die Verbraucher 62, 63, 64 umfasst, ansteuerbar beziehungsweise auswählbar und ansteuerbar ist.

Um die Übertragung der Signale von den Muskelimpulserfassungseinheiten 70 zu der Steuereinheiten 80 zu verdeutlichen, sind auch in der Figur 6 nur die Sendeeinheiten 75 der Muskelimpulserfassungseinheit 70 und die Empfangseinheit 81 der Steuereinheit 80 dargestellt.

Bei allen Ausführungsformen der Erfindung wurde die Tatsache, dass bei geeigneter Positionierung der wenigstens einen Muskelimpulserfassungseinheit 70 solche von der Sendeeinheit 75 der wenigstens einen Muskelimpulserfassungseinheit 70 gesendeten Signale von der Empfangseinheit 81 der wenigstens einen Steuereinheit 80 empfangen werden können, nur sehr stark schematisiert anhand von Pfeilen dargestellt. Mittels der Pfeile wird angedeutet, dass die Empfangseinheit 81 der wenigstens einen Steuereinheit 80 in dem räumlichen Sendebereich der Sendeeinheit der wenigstens einen Muskelimpulserfassungseinheit 70 positioniert oder positionierbar ist. Gleiches gilt im Wesentlichen für die Verbindung zwischen der Steuereinheit 80 einerseits und der Verbraucher 61, 62, 63, 64 andererseits, wie beispielsweise in Figuren 2 und 6 dargestellt, wobei diese Verbindung mittels drahtgebundener Kommunikationsmittel, wie insbesondere DALI oder POWERLINE, oder drahtloser Kommunikationsmittel, wie insbesondere Bluetooth, zur Übertragung der Steuerungsbefehle von der wenigstens einen Steuereinheit 80 an den wenigstens einen Verbraucher 61, 62, 63, 64 bereitgestellt werden kann.

Im Folgenden wird ein bevorzugtes Verfahren zum Ansteuern von Verbrauchern 61, 62, 63, 64 einer Hausleittechnik, insbesondere von Leuchten, mittels Muskelimpulsen wenigstens eines Benutzers beschrieben.

In einem ersten Schritt werden elektrische Muskelimpulse wenigstens eines Benutzers erfasst, die beim Durchführen einer oder mehrerer im freien Raum erzeugter Gesten entstehen. Dies geschieht mittels wenigstens einer Muskelimpulserfassungseinheit 70 in Form von einer Geste eindeutig zugeordneten vorzugsweise digitalen Signalen. In einem zweiten Schritt werden die den Gesten eindeutig zugeordneten Signale mittels einer Sendeeinheit 75 der Muskelimpulserfassungseinheit 70 übermittelt; also ausgegeben. In einem dritten Schritt werden die von der Sendeeinheit 75 der Muskelimpulserfassungseinheit 70 übermittelten Signale mittels einer Empfangseinheit 81 wenigstens einer Steuereinheit 80 empfangen. Die empfangenen Signale werden sodann in einem vierten Schritt zu in der Steuereinheit 80 hinterlegten Steuerungsbefehlen mittels einer Zuordnungseinheit 82 der Steuereinheit 80 zugeordnet. In einem fünften Schritt wird wenigstens ein Verbraucher 61, 62, 63, 64 der Hausleittechnik auf Basis der den empfangenen Signalen zugeordneten Steuerungsbefehle mittels einer Ansteuereinheit 83 der Steuereinheit 80 angesteuert. Es ist dabei denkbar, dass mit einer Muskelimpulserfassungseinheit 70 nur ein in der Steuereinheit 80 vordefinierter Verbraucher 61 oder eine vordefinierte Gruppe von Verbrauchern 62, 63, 64 oder auch alle Verbraucher 61, 62, 63, 64 angesteuert werden.

Handelt es sich bei den Verbrauchern um Leuchten eines Beleuchtungssystems, so können ein oder mehrere der Verbraucher 61, 62, 63, 64 durch die Steuerungsbefehle wenigstens eine als ein Verbraucher 61, 62, 63, 64 dienende Leuchte ein- oder ausgeschaltet werden und/oder die Farbtemperatur und/oder Helligkeit und/oder weitere Parameter der wenigstens einen jeweils als ein Verbraucher 61, 62, 63, 64 dienende Leuchte eingestellt werden können.

Das Verfahren kann ferner die Schritte aufweisen, dass eine vordefinierten Gruppe von empfangenen Signalen zu in der Steuereinheit 80 hinterlegten Auswahlbefehlen mittels der Zuordnungseinheit 82 der Steuereinheit 80 zugeordnet werden, wobei in einem folgenden Schritt ein anzusteuernder Verbraucher oder eine Gruppe von anzusteuernden Verbrauchern mittels einer Auswahleinheit 84 der Steuereinheit 80 auf Basis der den empfangenen Signalen der vordefinierten Gruppe zugeordneten Auswahlbefehle ausgewählt werden, um einzig die ausgewählten Verbraucher mittels der Steuerungsbefehle anzusteuern.

Vorzugsweise wird ein anzusteuernder Verbraucher 61, 62, 63, 64 mittels eines dem - vorzugsweise innerhalb einer vorbestimmten Zeit - zuletzt empfangenen digitalen Signal zugeordneten Steuerungsbefehls angesteuert.

Vorzugsweise kann ein empfangenes Signal einem Auswahlbefehl oder einem Steuerungsbefehl zugeordnet oder einem Auswahlbefehl oder einem Steuerungsbefehl ein empfangenes Signal zugeordnet und diese Zuordnung in der Speichereinheit 85 der Steuereinheit 80 hinterlegt werden.

Neben der voranstehenden schriftlichen Offenbarung wird hiermit zur weiteren Offenbarung der Erfindung ergänzend auf die Darstellung in den Figuren 2 bis 6 Bezug genommen.

Die Erfindung ist nicht auf die zuvor beschriebenen Ausführungsbeispiele beschränkt, solange sie vom Gegenstand der folgenden Ansprüche umfasst ist. Ferner sind die zuvor beschriebenen Ausführungsbeispiele sowie die darin beschriebenen Merkmale in beliebiger Weise in einem individuell bereitgestellten System miteinander kombinierbar. So kann bspw. ein System auch zentrale sowie dezentrale bzw. einem oder mehreren Verbrauchern direkt zugeordnete Steuereinheiten 80 aufweisen. Die Steuereinheit(en) 80 eines Systems kann/können je einem Verbraucher 61, 62, 63, 64, einer Gruppe von Verbrauchern und/oder allen Verbrauchern 61, 62, 63, 64 des Systems 50, 51, 52 zugeordnet sein, um den ihr zugeordnete Verbraucher 61, 62, 63, 64 oder die ihr zugeordneten Verbraucher 61, 62, 63, 64 wahlweise anzusteuern. Die Steuereinheit 80 kann hierzu wahlweise als Zentralsteuereinheit, separate Steuereinheit oder Teil des Verbrauchers 61, 62, 63, 64 ausgebildet sein.

Ebenso können in einem System Muskelimpulserfassungseinheiten 70 sowohl mit beschränktem als auch mit Voll-Zugriff verwendet werden. Die Beschränkung kann dabei beliebig festgelegt und wahlweise auch änderbar sein/ geändert werden. Ebenso ist die Erfindung nicht auf bestimmte Gesten beschränkt. Vielmehr kann beliebigen Gesten oder Gestenkombinationen ein gewünschter Befehl zugeordnet und abgespeichert werden. Dabei umfasst der Begriff Geste alles, was mittels der Muskelimpulserfassungseinheit 70 messbare Muskelimpulse auslösen kann.

### Bezugszeichenliste

- 10: Arm
- 20: Hand
- 21: Handinnenfläche
- 31: Daumen
- 32: Zeigefinger
- 33: Mittelfinger
- 35: kleiner Finger
- 40: Unterarmmuskeln
- 50, 51, 52: jeweils ein erfindungsgemäßes System zum Ansteuern wenigstens eines Verbrauchers einer Hausleittechnik
- 61, 62, 63, 64: Verbraucher einer Hausleittechnik
- 70: Muskelimpulserfassungseinheit
- 71, 72: Sensoren
- 75: Sendeeinheit
- 80: Steuereinheit
- 81: Empfangseinheit
- 82: Zuordnungseinheit
- 83: Ansteuereinheit
- 84: Auswahleinheit
- 85: Speichereinheit

## Patentansprüche

1. System (50, 51, 52) zum Ansteuern von Verbrauchern (61, 62, 63, 64) einer Haushaltsleittechnik, insbesondere von Leuchten, mittels Muskelimpulsen wenigstens eines Benutzers, aufweisend:
- wenigstens eine Muskelimpulserfassungseinheit (70) zum Erfassen von elektrischen Muskelimpulsen, die beim Durchführen einer oder mehrerer im freien Raum erzeugten Gesten entstehen, in Form von einer Geste eindeutig zugeordneten vorzugsweise digitalen Signalen, wobei die Muskelimpulserfassungseinheit (70) ferner eine Sendeeinheit (75) zum Übermitteln der den Gesten eindeutig zugeordneten Signale aufweist,
- wenigstens eine Steuereinheit(80) aufweisend:
∘ eine Empfangseinheit (81) zum Empfangen der von der Sendeeinheit (75) übermittelten Signale,
∘ eine Zuordnungseinheit (82) zum Zuordnen der empfangenen Signale zu in der Steuereinheit (80) hinterlegten Steuerungsbefehlen, und
∘ eine Ansteuereinheit (83) zum Ansteuern wenigstens eines der Verbraucher (61, 62, 63, 64) der Hausleittechnik auf Basis der den empfangenen Signalen zugeordneten Steuerungsbefehlen,
das System (50, 51, 52) ist **dadurch gekennzeichnet, dass** die Zuordnungseinheit (82) ferner dazu ausgebildet ist, eine vordefinierte Gruppe von Signalen zu in der Steuereinheit (80) hinterlegten Auswahlbefehlen zuzuordnen, wobei die Steuereinheit (80) ferner eine Auswahleinheit (84) aufweist, die ausgebildet ist, auf Basis der den empfangenen Signalen der vordefinierten Gruppe zugeordneten Auswahlbefehlen einen oder eine Gruppe von anzusteuernden Verbrauchern auszuwählen, um einzig die ausgewählten Verbraucher mittels der Steuerungsbefehle anzusteuern.

2. System (50, 51, 52) nach Anspruch 1, ferner aufweisend wenigstens einen Verbraucher (61, 62, 63, 64), insbesondere wenigstens eine Leuchten, welcher mittels der Steuerungsbefehle ansteuerbar ist,
wobei die Steuereinheit (80) mit dem wenigstens einen Verbraucher (61, 62, 63, 64) vorzugsweise mittels drahtgebundener Kommunikationsmittel, wie insbesondere DALI oder POWERLINE, oder drahtloser Kommunikationsmittel, wie insbesondere Bluetooth, zur Übertragung der Steuerungsbefehle von der wenigstens einen Steuereinheit (80) an den wenigstens einen Verbraucher (61, 62, 63, 64) verbunden ist.

3. System (50, 51, 52) nach einem der vorhergehenden Ansprüche, **dadurch gekennzeichnet, dass** die Sendeeinheit (75) der Muskelimpulserfassungseinheit (70) und die Empfangseinheit (81) der Steuereinheit (80) drahtlose Kommunikationsmittel aufweisen, vorzugsweise Bluetooth-Module, zur drahtlosen Signalübertragung.

4. System (50, 51) nach einem der vorhergehenden Ansprüche, **dadurch gekennzeichnet, dass**
die Steuereinheit (80) dazu ausgebildet ist, wenigstens einen anzusteuernden Verbraucher (61, 62, 63, 64) mittels eines dem - vorzugsweise innerhalb einer vorbestimmten Zeit - zuletzt empfangenen Signal zugeordneten Steuerungsbefehls anzusteuern, und/oder
die Steuereinheit (80) derart ausgebildet ist, dass mit einer Muskelimpulserfassungseinheit (70) nur ein vordefinierter Verbraucher (61) oder eine vordefinierte Gruppe von Verbrauchern (62, 63, 64) ansteuerbar ist.

5. System (50, 51, 52) nach einem der vorhergehenden Ansprüche, **dadurch gekennzeichnet, dass** die Steuereinheit (80) eine Speichereinheit (85) aufweist, die dazu ausgebildet ist, ein empfangenes Signal einem Auswahlbefehl und/oder einem Steuerungsbefehl zuzuordnen oder einem Auswahlbefehl und/oder einem Steuerungsbefehl ein empfangenes Signal zuzuordnen und diese Zuordnung in der Speichereinheit (85) zu hinterlegen.

6. System (50, 51, 52) nach einem der vorhergehenden Ansprüche, **dadurch gekennzeichnet, dass** eine Steuereinheit (80) je einem Verbraucher (61, 62, 63, 64), einer Gruppe von Verbrauchern oder allen Verbrauchern (61, 62, 63, 64) des Systems (50, 51, 52) zugeordnet ist, um den ihr zugeordnete Verbraucher (61, 62, 63, 64) oder die ihr zugeordneten Verbraucher (61, 62, 63, 64) wahlweise anzusteuern, wobei die Steuereinheit (80) als Zentralsteuereinheit, separate Steuereinheit oder Teil des Verbrauchers 61, 62, 63, 64) ausgebildet ist.

7. System (50, 51, 52) nach einem der vorhergehenden Ansprüche, **dadurch gekennzeichnet, dass** die Muskelimpulserfassungseinheit (70) Sensoren (71, 72) zur Erfassung von Muskelimpulsen aufweist und insbesondere als Armband mit diesen Sensoren ausgebildet ist.

8. System (50, 51, 52) nach einem der vorhergehenden Ansprüche, wobei die Steuerungsbefehle das Ein- und Ausschalten und/oder die Einstellung einer Farbtemperatur, Helligkeit und/oder weiterer Parameter wenigstens einer jeweils als ein Verbraucher (61, 62, 63, 64) dienenden Leuchte umfassen.

9. Verfahren zum Ansteuern von Verbrauchern (61, 62, 63, 64) einer Hausleittechnik, insbesondere von Leuchten, mittels Muskelimpulsen wenigstens eines Benutzers, aufweisend die folgenden Schritte:
- Erfassen von elektrischen Muskelimpulsen wenigstens eines Benutzers, die beim Durchführen einer oder mehrerer im freien Raum erzeugten Gesten entstehen, mittels wenigstens einer Muskelimpulserfassungseinheit (70) in Form von einer Geste eindeutig zugeordneten vorzugsweise digitalen Signalen,
- Übermitteln der den Gesten eindeutig zugeordneten Signale mittels einer Sendeeinheit (75) der Muskelimpulserfassungseinheit (70),
- Empfangen der von der Sendeeinheit (75) der Muskelimpulserfassungseinheit (70) übermittelten Signale mittels einer Empfangseinheit (81) wenigstens einer Steuereinheit (80),
- Zuordnen der empfangenen Signale zu in der Steuereinheit (80) hinterlegten Steuerungsbefehlen mittels einer Zuordnungseinheit (82) der Steuereinheit (80),
- Ansteuern wenigstens eines Verbrauchers (61, 62, 63, 64) der Hausleittechnik auf Basis der den empfangenen Signalen zugeordneten Steuerungsbefehle mittels einer Ansteuereinheit (83) der Steuereinheit (80),
**dadurch gekennzeichnet, dass** das Verfahren die folgenden Schritte aufweist:
- Zuordnen einer vordefinierten Gruppe von empfangenen Signalen zu in der Steuereinheit (80) hinterlegten Auswahlbefehlen mittels der Zuordnungseinheit (82) der Steuereinheit (80),
- Auswählen eines anzusteuernden Verbrauchers oder einer Gruppe von anzusteuernden Verbrauchern mittels einer Auswahleinheit (84) der Steuereinheit (80) auf Basis der den empfangenen Signalen der vordefinierten Gruppe zugeordneten Auswahlbefehle, um einzig die ausgewählten Verbraucher mittels der Steuerungsbefehle anzusteuern.

10. Verfahren nach Anspruch 9, **dadurch gekennzeichnet, dass** wenigstens ein anzusteuernder Verbraucher (61, 62, 63, 64) mittels eines dem - vorzugsweise innerhalb einer vorbestimmten Zeit - zuletzt empfangenen digitalen Signal zugeordneten Steuerungsbefehls angesteuert wird.

11. Verfahren nach einem der Ansprüche 9 oder 10, **dadurch gekennzeichnet, dass** mit einer Muskelimpulserfassungseinheit (70) nur ein in der Steuereinheit (80) vordefinierter Verbraucher (61) oder eine vordefinierte Gruppe von Verbrauchern (62, 63, 64) angesteuert wird, und/oder
beim Schritt des Ansteuerns eines oder mehrerer Verbraucher (61, 62, 63, 64) durch die Steuerungsbefehle wenigstens einer als ein Verbraucher (61, 62, 63, 64) dienenden Leuchte ein- oder ausgeschaltet wird und/oder die Farbtemperatur und/oder Helligkeit und/oder weitere Parameter der wenigstens einen jeweils als ein Verbraucher 61, 62, 63, 64) dienenden Leuchte eingestellt werden.

12. Verfahren nach einem der Ansprüche 9 bis 11, **dadurch gekennzeichnet, dass** ein empfangenes Signal einem Auswahlbefehl oder einem Steuerungsbefehl zugeordnet oder einem Auswahlbefehl oder einem Steuerungsbefehl ein empfangenes Signal zugeordnet und diese Zuordnung in einer Speichereinheit (85) der Steuereinheit (80) hinterlegt wird.

13. Verfahren nach einem der Ansprüche 9 bis 12, **dadurch gekennzeichnet, dass** die im freien Raum erzeugten Gesten ein Wischen mit einer Hand (20) entlang einer ersten Richtung und/oder ein Wischen mit einer Hand (20) entlang einer der ersten Richtung entgegengesetzten Richtung und/oder ein Öffnen einer Faust und/oder ein Schließen einer Faust und/oder ein Bewegen eines Armes (10) entlang einer gegenüber der ersten Richtung senkrecht verlaufenden zweiten Richtung und/oder ein Bewegen eines Armes (10) entlang einer der zweiten Richtung entgegengesetzten Richtung und/oder ein Rotieren eines Armes (10) in einer ersten Rotationsrichtung und/oder ein Rotieren eines Armes (10) in einer der ersten Rotationsrichtung entgegengesetzten Rotationrichtung und/oder ein Fingerschnippen und/oder ein Öffnen einzelner Finger einer Hand (20) und/oder ein Schließen einzelner Finger einer Hand (20) und/oder ein Öffnen einer ersten Anzahl von Fingern (31, 32, 33) einer Hand (20), deren Handinnenfläche (21) in die erste Richtung zeigt, und/oder ein Öffnen einer zweiten Anzahl von Fingern (32, 33) einer Hand (20), deren Handinnenfläche (21) in die der zweiten Richtung entgegengesetzten Richtung zeigt, umfassen.

## Claims

1. A system (50, 51, 52) for actuating loads (61, 62, 63, 64) of a household control technology, in particular of lamps, by means of muscle impulses of at least one user, having:
- At least one muscle-impulse detection unit (70) for detecting electrical muscle impulses, which are produced when one or more gestures generated in free space are performed, in the form of preferably digital signals uniquely associated with a gesture, wherein the muscle-impulse detection unit (70) also has a transmitting unit (75) for transmitting the signals uniquely associated with the gestures,
- at least one control unit (80) having:
∘ A receiving unit (81) for receiving the signals transmitted by the transmitting unit (75),
∘ an associating unit (82) for associating the received signals with control commands stored in the control unit (80), and
∘ an actuating unit (83) for actuating at least one of the loads (61, 62, 63, 64) of the household control technology on the basis of the control commands associated with the received signals,
the system (50, 51, 52) is **characterized in that**
the associating unit (82) is also designed to associate a pre-defined group of signals with selection commands stored in the control unit (80),
wherein the control unit (80) also has a selection unit (84), which is designed to select one or a group of loads to be actuated on the basis of the selection commands associated with the received signals of the pre-defined group, in order to uniquely actuate the selected loads by means of the control commands.

2. The system (50, 51, 52) according to Claim 1, also having at least one load (61, 62, 63, 64), in particular at least one lamp, which can be actuated by means of the control commands,
wherein the control unit (80) is connected to the at least one load (61, 62, 63, 64) preferably by means of wired communication means, such as in particular DALI or POWERLINE, or wireless communication means, such as in particular Bluetooth, for transmitting the control commands from the at least one control unit (80) to the at least one load (61, 62, 63, 64).

3. The system (50, 51, 52) according to any one of the preceding claims, **characterized in that** the transmitting unit (75) of the muscle-impulse detection unit (70) and the receiving unit (81) of the control unit (80) have wireless communication means, preferably Bluetooth modules, for the wireless signal transmission.

4. The system (50, 51) according to any one of the preceding claims, **characterized in that** the control unit (80) is designed, to actuate at least one load (61, 62, 63, 64) to be actuated by means of a control command associated with the - preferably within a predetermined time - last received signal, and/or
the control unit (80) is designed is such a manner that only one pre-defined load (61) or a pre-defined group of loads (62, 63, 64) can be actuated with a muscle-impulse detection unit (70).

5. The system (50, 51, 52) according to any one of the preceding claims, **characterized in that** the control unit (80) has a memory unit (85), which is designed to associate a received signal with a selection command and/or a control command or to associate a received signal with a selection command and/or a control command and to store this association in the memory unit (85).

6. The system (50, 51, 52) according to any one of the preceding claims, **characterized in that** a control unit (80) is associated with each load (61, 62, 63, 64), a group of loads or all loads (61, 62, 63, 64) of the system (50, 51, 52), in order to selectively actuate the load (61, 62, 63, 64) associated with it or the loads (61, 62, 63, 64) associated with it, wherein the control unit (80) is designed as a central control unit, separate control unit or part of the load (61, 62, 63, 64).

7. The system (50, 51, 52) according to any one of the preceding claims, **characterized in that** the muscle-impulse detection unit (70) has sensors (71, 72) for detecting muscle impulses and in particular is designed as a wrist band with said sensors.

8. The system (50, 51, 52) according to any one of the preceding claims, wherein the control commands comprise a switching on and off and/or the setting of a color temperature, brightness and/or further parameters at least of one lamp serving in each case as a load (61, 62, 63, 64).

9. A method for actuating loads (61, 62, 63, 64) of a household control technology, in particular of lamps, by means of muscle impulses at least of one user, having the following steps:
- Detection of electrical muscle impulses at least of one user, which are produced when one or more gestures generated in free space are performed, by means of at least one muscle-impulse detection unit (70) in the form of preferably digital signals uniquely associated with a gesture,
- transmission of the signals uniquely associated with the gestures by means of a transmitting unit (75) of the muscle-impulse detection unit (70),
- reception of the signals transmitted by the transmitting unit (75) of the muscle-impulse detection unit (70) by means of a receiving unit (81) at least of one control unit (80),
- association of the received signals with the control commands stored in the control unit (80) by means of an associating unit (82) of the control unit (80),
- actuation of at least one load (61, 62, 63, 64) of the household control technology on the basis of the control commands associated with the received signals by means of an actuating unit (83) of the control unit (80),
**characterized in that** the method has the following steps:
- Association of a pre-defined group of received signals with selection commands stored in the control unit (80) by means of the associating unit (82) of the control unit (80)
- selection of a load to be actuated or a group of loads to be actuated by means of a selection unit (84) of the control unit (80) on the basis of the selection commands associated with the received signals of the pre-defined group, in order to uniquely actuate the selected loads by means of the control commands.

10. The method according to Claim 9, **characterized in that** at least one load (61, 62, 63, 64) to be actuated is actuated by means of a control command associated with the - preferably within a predetermined time - last received digital signal.

11. The method according to any one of Claims 9 or 10, **characterized in that** only one load (61) pre-defined in the control unit (80) or a pre-defined group of loads (62, 63, 64) is actuated with a muscle-impulse detection unit (70) and/or
in the step of actuating one or more loads (61, 62, 63, 64) by the control commands at least one lamp serving as a load (61, 62, 63, 64) is switched on or off and/or the color temperature and/or brightness and/or further parameters of the at least one lamp serving in each case as a load (61, 62, 63, 64) are set.

12. The method according to any one of Claims 9 to 11, **characterized in that** a received signal is associated with a selection command or a control command or a received signal is associated with a selection command or a control command and this association is stored in a memory unit (85) of the control unit (80).

13. The method according to any one of Claims 9 to 12, **characterized in that** the gestures generated in free space comprise a swiping with a hand (20) along a first direction and/or a swiping with a hand (20) along a direction opposite to the first direction and/or an opening of a fist and/or a closing of a fist and/or a movement of an arm (10) along a second direction running perpendicular relative to the first direction and/or a movement of an arm (10) along a direction opposite to the second direction and/or a rotation of an arm (10) in a first rotational direction and/or a rotation of an arm (10) in a rotational direction opposite to the first rotational direction and/or a snapping of a finger and/or an opening of individual fingers of a hand (20) and/or a closing of individual fingers of a hand (20) and/or an opening of a first number of fingers (31, 32, 33) of a hand (20), the palm (21) of which points in the first direction, and/or an opening of a second number of fingers (32, 33) of a hand (20), the palm (21) of which points in the direction opposite to the second direction.

## Revendications

1. Système (50, 51, 52) pour le contrôle de consommateurs (61, 62, 63, 64) d'un système domotique, plus particulièrement de luminaires, au moyen d'impulsions musculaires d'au moins un utilisateur, comprenant :
- au moins une unité de détection d'impulsions musculaires (70) pour la détection d'impulsions musculaires électriques qui apparaissent lors de la réalisation d'un ou de plusieurs gestes effectués à l'air libre, sous la forme de signaux, de préférence numériques, attribués de manière unique à un geste, l'unité de détection d'impulsions musculaires (70) comprenant en outre une unité d'émission (75) pour la transmission des signaux attribués de manière unique aux gestes,
- au moins une unité de commande (80) comprenant :
• une unité de réception (81) pour la réception des signaux transmis par l'unité d'émission (75),
• une unité d'attribution (82) pour l'attribution des signaux reçus à des instructions de commande enregistrées dans l'unité de commande (80) et
• une unité de pilotage (83) pour le contrôle d'au moins un des consommateurs (61, 62, 63, 64) du système domotique sur la base des instructions de commande attribuées aux signaux reçus,
le système (50, 51, 52) étant **caractérisé en ce que** l'unité d'attribution (82) est en outre conçue pour attribuer un groupe prédéfini de signaux à des instructions de sélection enregistrées dans l'unité de commande (80), l'unité de commande (80) comprenant en outre une unité de sélection (84) qui est conçue pour sélectionner, sur la base des instructions de sélection attribuées aux signaux reçus du groupe prédéfini, un consommateur ou un groupe de consommateurs à piloter, afin de contrôler de manière unique les consommateurs sélectionnés au moyen des instructions de commande.

2. Système (50, 51, 52) selon la revendication 1, comprenant en outre au moins un consommateur (61, 62, 63, 64), plus particulièrement au moins un luminaire, qui peut être contrôlé au moyen des instructions de commande,
l'unité de commande (80) étant reliée avec l'au moins un consommateur (61, 62, 63, 64) de préférence à l'aide de moyens de communication filaires, plus particulièrement DALI ou POWERLINE, ou de moyens de communication sans fil, plus particulièrement Bluetooth, pour la transmission des instructions de commande par l'au moins une unité de commande (80) à l'au moins un consommateur (61, 62, 63, 64).

3. Système (50, 51, 52) selon l'une des revendications précédentes, **caractérisé en ce que** l'unité d'émission (75) de l'unité de détection d'impulsions musculaires (70) et l'unité de réception (81) de l'unité de commande (80) comprennent des moyens de communication filaires, de préférence des modules Bluetooth, pour la transmission de signaux sans fil.

4. Système (50, 51) selon l'une des revendications précédentes, **caractérisé en ce que**
l'unité de commande (80) est conçue pour contrôler au moins un consommateur (61, 62, 63, 64) à contrôler au moyen d'une instruction de commande attribuée au signal reçu en dernier, de préférence dans un temps prédéterminé, et/ou
l'unité de commande (80) est conçue de façon à ce que, avec une unité de détection d'impulsions musculaires (70), un seul consommateur (61) prédéfini ou un groupe prédéfini de consommateurs (62, 63, 64) soit contrôlable.

5. Système (50, 51, 52) selon l'une des revendications précédentes, **caractérisé en ce que** l'unité de commande (80) comprend une unité de mémoire (85) qui est conçue pour attribuer un signal reçu à une instruction de sélection et/ou à une instruction de commande ou pour attribuer, à une instruction de sélection et/ou à une instruction de commande, un signal reçu et pour enregistrer cette attribution dans l'unité de mémoire (85).

6. Système (50, 51, 52) selon l'une des revendications précédentes, **caractérisé en ce qu'**une unité de commande (80) est attribuée à chaque consommateur (61, 62, 63, 64), à un groupe de consommateurs ou à tous les consommateurs (61, 62, 63, 64) du système (50, 51, 52), afin de contrôler de manière sélective le consommateur (61, 62, 63, 64) qui lui est attribué ou les consommateurs (61, 62, 63, 64) qui lui sont attribués, l'unité de commande (80) étant conçue comme une unité de commande centrale, une unité de commande séparée ou une partie du consommateur (61, 62, 63, 64).

7. Système (50, 51, 52) selon l'une des revendications précédentes, **caractérisé en ce que** l'unité de détection d'impulsions musculaires (70) comprend des capteurs (71, 72) pour la détection d'impulsions musculaires et plus particulièrement est conçue comme un bracelet avec ces capteurs.

8. Système (50, 51, 52) selon l'une des revendications précédentes, les instructions de commande comprenant la mise en marche et l'arrêt et/ou le réglage d'une température de couleur, de la luminosité et/ou d'autres paramètres d'au moins un luminaire servant de consommateur (61, 62, 63, 64).

9. Procédé de contrôle de consommateurs (61, 62, 63, 64) d'un système domotique, plus particulièrement de luminaires, au moyen d'impulsions musculaires d'au moins un utilisateur, comprenant les étapes suivantes :
- détection d'impulsions musculaires électriques d'au moins un utilisateur, qui apparaissent lors de la réalisation d'un ou de plusieurs gestes effectués à l'air libre, sous la forme de signaux, de préférence numériques, attribués de manière unique à un geste, l'unité de détection d'impulsions musculaires (70) comprenant en outre une unité d'émission (75) pour la transmission des signaux attribués de manière unique aux gestes,
- transmission des signaux attribués de manière unique aux gestes au moyen d'une unité d'émission (75) de l'unité de détection d'impulsions musculaires (70),
- réception des signaux transmis par l'unité d'émission (75) de l'unité de détection d'impulsions musculaires (70) au moyen d'une unité de réception (81) d'au moins une unité de commande (80),
- attribution des signaux reçus à des instructions de commande enregistrées dans l'unité de commande (80) au moyen d'une unité d'attribution (82) de l'unité de commande (80),
- contrôle d'au moins un consommateur (61, 62, 63, 64) du système domotique sur la base des instructions de commande attribuées aux signaux reçus au moyen d'une unité de pilotage (83) de l'unité de commande (80),
**caractérisé en ce que** le procédé comprend les étapes suivantes :
- attribution d'un groupe prédéfini de signaux reçus à des instructions de sélection enregistrées dans l'unité de commande (80) au moyen de l'unité d'attribution (82) de l'unité de commande (80),
- sélection d'un consommateur à contrôler ou d'un groupe de consommateurs à contrôler au moyen d'une unité de sélection (84) de l'unité de commande (80) sur la base des instructions de commande attribuées aux signaux reçus du groupe prédéfini, afin de contrôler de manière unique les consommateurs sélectionnés au moyen des instructions de commande.

10. Procédé selon la revendication 9, **caractérisé en ce qu'**au moins un consommateur (61, 62, 63, 64) à contrôler est contrôlé au moyen d'une instruction de commande attribuée au signal numérique reçu en dernier, de préférence dans un temps prédéfini.

11. Procédé selon l'une des revendications 9 ou 10, **caractérisé en ce que**, avec une unité de détection d'impulsions musculaires (70), seul un consommateur (61) prédéfini dans l'unité de commande (80) ou un groupe prédéfini de consommateurs (62, 63, 64) est contrôlé et/ou
lors de l'étape du contrôle d'un ou plusieurs consommateurs (61, 62, 63, 64) par les instructions de commande, au moins un luminaire servant de consommateur (61, 62, 63, 64) est mis en marche ou éteint et/ou la température de couleur et/ou la luminosité et/ou d'autres paramètres de l'au moins un luminaire servant de consommateur (61, 62, 63, 64) est réglé.

12. Procédé selon l'une des revendications 9 à 11, **caractérisé en ce qu'**un signal reçu est attribué à une instruction de sélection ou à une instruction de commande ou, à une instruction de sélection ou à une instruction de commande, est attribué un signal reçu et cette attribution est enregistrée dans une unité de mémoire (85) de l'unité de commande (80).

13. Procédé selon l'une des revendications 9 à 12, **caractérisé en ce que** les gestes effectués à l'air libre comprennent un balayage avec une main (20) le long d'une première direction et/ou un balayage avec une main (20) le long d'une direction opposée à la première direction et/ou l'ouverture d'un poing et/ou la fermeture d'un poing et/ou le déplacement d'un bras (10) le long d'une deuxième direction s'étendant perpendiculairement à la première direction et/ou le déplacement d'un bras (10) le long d'une direction opposée à la deuxième direction et/ou la rotation d'un bras (10) dans un premier sens de rotation et/ou la rotation d'un bras (10) dans un sens de rotation opposé au premier sens de rotation et/ou un claquement de doigts et/ou l'ouverture de certains doigts d'une main (20) et/ou la fermeture de certains doigts d'une main (20) et/ou l'ouverture d'un premier nombre de doigts (31, 32, 33) d'une main (20), dont la paume (21) est orientée dans la première direction et/ou l'ouverture d'un deuxième nombre de doigts (32, 33) d'une main (20), dont la paume (21) est orientée dans la direction opposée à la deuxième direction.
